# EUROPÄISCHE PATENTSCHRIFT

(11) **EP 3 873 389 B1**
(45) Veröffentlichungstag und Bekanntmachung des Hinweises auf die Patenterteilung: **25.12.2024**
(21) Anmeldenummer: 19790164.8
(22) Anmeldetag: 14.10.2019
(51) Int. Cl.: A61F 5/01

(54) **KNIEORTHESE**
KNEE BRACE
ORTHÈSE DE GENOU

(30) Priorität: 29.10.2018 DE 102018126990
(43) Veröffentlichungstag der Anmeldung: 08.09.2021
(73) Patentinhaber: Ottobock SE & Co. KGaA, 37115 Duderstadt (DE)
(72) Erfinder: SIEWERT, Gordon, 37083 Göttingen (DE); SCHMITT, Alexander, 34123 Kassel (DE); SCHILLING, Matthias, 37345 Weißenborn-Lüderode (DE)
(74) Vertreter: Gramm, Lins & Partner Patent- und Rechtsanwälte PartGmbB
(86) Internationale Anmeldenummer: PCT/EP2019/077819
(87) Internationale Veröffentlichungsnummer: WO 2020/088925

(56) Entgegenhaltungen:
- EP-A1- 0 297 766
- EP-A2- 3 378 448
- EP-B1- 0 297 766
- WO-A1-2008/104149
- WO-A1-95/32692
- US-A1- 2009 240 181

## Beschreibung

Die Erfindung betrifft eine Knieorthese mit einem Unterschenkelteil, das sich in einer Unterschenkelrichtung erstreckt, und einem Oberschenkelteil, das sich in einer Oberschenkelrichtung erstreckt, und wenigstens einer Arretierungseinrichtung, wobei das Unterschenkelteil und das Oberschenkelteil durch wenigstens ein Schwenkgelenk schwenkbar miteinander verbunden sind, das Unterschenkelteil und/oder das Oberschenkelteil eine Lateralschiene und eine Medialschiene aufweist, und die Unterschenkelrichtung mit der Oberschenkelrichtung einen Kniefrontalwinkel einschließt, wobei die Knieorthese ein erstes Aufnahmeelement und ein zweites Aufnahmeelement aufweist, die in verschiedenen Positionen relativ zueinander mittels der wenigstens einen Arretierungseinrichtung festlegbar sind.

Knieorthesen sind in unterschiedlichsten Ausführungsformen aus dem Stand der Technik bekannt und werden beispielsweise verwendet, um eine Osteoarthritis im Knie zu behandeln. Zudem kann sie verwendet werden, um Kniefehlstellungen, insbesondere "genu varum" und "genu valgum" zu behandeln. Diese Fehlstellungen unterscheiden sich im Kniefrontalwinkel des Beines, also des Winkels zwischen der Längsrichtung des Oberschenkels und der Längsrichtung des Unterschenkels des Beines, der in der Frontalebene liegt. Eine Orthese für diese Anwendungsfälle verfügt ebenfalls über einen Kniefrontalwinkel zwischen der Oberschenkelrichtung und der Unterschenkelrichtung. Auch dieser Kniefrontalwinkel liegt in der Frontalebene.

Eine gattungsgemäße Knieorthese ist beispielsweise aus der US 6,981,957 B2 bekannt. Derartige Orthese sollten in der Lage sein, sowohl für "genu varum"-Fehl-stellungen als auch für "genu valgum", also "O"- und "X"-Beine verwendet zu werden. Dazu hat es sich als vorteilhaft herausgestellt, wenn ein Winkel zwischen dem Unterschenkelteil und dem Oberschenkelteil einstellbar ausgebildet ist. Das Unterschenkelteil wird verwendet, um am Unterschenkel des Patienten befestigt zu werden, während das Oberschenkelteil am Oberschenkel befestigt wird. Da es sich beim Träger um einen Patienten mit einer Kniefehlstellung handelt, verlaufen Unterschenkel und Oberschenkel nicht parallel, sondern in einem Winkel zueinander, der durch die Knieorthese abgebildet werden muss. Um nicht für jeden Winkel eine separate Orthese anfertigen zu müssen oder um eine große Anzahl unterschiedlicher Knieorthesen vorzuhalten, ist aus dem Stand der Technik bekannt, diesen Winkel einstellbar auszugestalten. Dies wird bei der gattungsgemäßen Orthese aus dem Stand der Technik dadurch erreicht, dass die Lateralschiene und die Medialschiene längenveränderlich ausgebildet sind und über ein Frontalgelenk verfügen, während das Schwenkgelenk, mit dem Oberschenkelteil und Unterschenkelteil miteinander verbunden sind, eine Verschwenkung um die Knieachse und damit eine natürliche Bewegung des Knies ermöglicht, weisen die Frontalgelenke aus dem Stand der Technik eine Schwenkachse auf, die senkrecht zu dieser Richtung verläuft. Auf diese Weise lässt sich durch die Längenveränderbarkeit der Medialschiene und der Lateralschiene ein Winkel zwischen dem Oberschenkelteil und dem Unterschenkelteil und auf die persönlichen Bedürfnisse des Trägers anpassen. Nachteilig ist, dass die Vorrichtung aus dem Stand der Technik konstruktiv aufwendig und damit kostenintensiv ist.

Aus der WO 2008/104149 A1, die den Oberbegriff des Anspruchs 1 offenbart, ist eine Knieorthese bekannt, bei der der Unterschenkelteil zwei proximale Schenkel aufweist, die um eine Schwenkachse gegeneinander verschwenkt und in unterschiedlichen Orientierungen zueinander festgelegt werden können. Eine Änderung der Position der beiden Schenkel relativ zueinander findet nicht statt.

Auch aus der WO 95/32692 A1 und der US 2009/240181 A1 sind Knieorthesen bekannt, deren Oberschenkel- und Unterschenkelrahmen verstellbar ausgebildet sind. Dabei lässt sich die Breite der einzelnen Elemente einstellen. Auch die EP 3 378 448 A2 beschreibt eine Orthese, bei der die Weite des Oberschenkelrahmens und des Unterschenkelrahmens eingestellt werden kann.

Der Erfindung liegt daher die Aufgabe zugrunde, eine Knieorthese so weiterzuentwickeln, dass sie konstruktiv einfach herzustellen ist und dennoch für unterschiedlichste Kniefehlstellungen verwendet werden kann.

Die Erfindung löst die gestellte Aufgabe durch eine Knieorthese gemäß dem Anspruch 1.

In den verschiedenen Positionen, in denen das erste Aufnahmeelement und das zweite Aufnahmeelement aneinander festgelegt werden können, weist die Knieorthese unterschiedliche Kniefrontalwinkel auf. Es verändert sich je nach Position also der Winkel zwischen der Unterschenkelrichtung und der Oberschenkelrichtung. Die Unterschenkelrichtung ist dabei vorzugsweise die Richtung, in der sich der Unterschenkel des Trägers der Knieorthese erstreckt, wenn die Knieorthese bestimmungsgemäß getragen wird. Die Oberschenkelrichtung ist dabei vorzugsweise entsprechend die Richtung, in der sich der Oberschenkel des Trägers der Knieorthese erstreckt, wenn die Knieorthese bestimmungsgemäß getragen wird. Um von einer Position, in der das erste Aufnahmeelement und das zweite Aufnahmeelement aneinander befestigt sind oder sein können, in eine zweite davon verschiedene Position zu gelangen, ist es nicht ausreichend, die beiden Aufnahmeelemente relativ zueinander zu verschwenken. Dies würde nicht die Position der beiden Aufnahmeelemente relativ zueinander, sondern lediglich die Orientierung der beiden Elemente zueinander verändern.

Vorzugsweise sind das Unterschenkelteil und das Oberschenkelteil durch zwei Schwenkgelenke schwenkbar mit einander verbunden. Eines der Schwenkgelenke ist vorzugsweise lateral und eines medial angeordnet. Vorzugsweise sind die beiden Schwenkachsen der beiden Schwenkgelenke koaxial angeordnet. Die verschiedenen Kniefrontalwinkel entstehen bevorzugt dadurch, dass ein Verhältnis der Abstände zwischen dem Kontaktbereich, in dem die beiden Aufnahmeelemente an einander anliegen oder an einander befestigt sind, und den beiden Gelenken, die den Unterschenkelteil mit dem Oberschenkelteil verbinden, verändert wird.

Die Aufnahmeelemente können beispielsweise in Form von Halbschalen oder schalenförmigen Elementen ausgebildet sein und im aneinander befestigten Zustand den Oberschenkel oder den Unterschenkel des Patienten vollständig umfassen. Im Unterschied zum Stand der Technik, der an dieser Stelle Verbindungsstangen zwischen der Medialschiene und der Lateralschiene vorsieht, die die beiden Schienen dauerhaft miteinander verbinden, ist erfindungsgemäß vorgesehen, zwischen der Lateralschiene und der Medialschiene das erste Aufnahmeelement und das zweite Aufnahmeelement vorzusehen, die lösbar miteinander verbunden oder lösbar miteinander verbindbar sind. Durch die Arretierungseinrichtung können sie aneinander festgelegt werden. Dies bedeutet, dass zum Verändern der Position des lateralen Aufnahmeelementes relativ zum medialen Aufnahmeelement die Arretierungseinrichtung gelöst werden muss.

Die verschiedenen Positionen, in denen die Aufnahmeelemente relativ zueinander befestigt werden können, sind vorteilhafterweise stufenlos einstellbar. Alternativ dazu sind verschiedene diskrete, also voneinander beabstandete, Positionen vorgesehen, in denen die beiden Aufnahmeelemente relativ zueinander positioniert und durch die Arretierungseinrichtung festlegbar sind. In unterschiedlichen Positionen kommt durch die vorteilhafterweise feste Verbindung des lateralen Aufnahmeelementes mit der Lateralschiene und des medialen Aufnahmeelementes mit der medialen Schiene zu einer Veränderung des Winkels zwischen dem Unterschenkelteil und dem Oberschenkelteil, wodurch unterschiedlichen Kniefehlstellungen Rechnung getragen werden kann.

In einer bevorzugten Ausgestaltung ist das erste Aufnahmeelement an der Lateralschiene und das zweite Aufnahmeelement an der Medialschiene befestigt. Bevorzugt ist das erste Aufnahmeelement dabei nur an der Lateralschiene und nicht an der Medialschiene und das zweite Aufnahmeelement nur an der Medialschiene und nicht an der Lateralschiene befestigt.

Vorzugsweise sind das erste Aufnahmeelement und das zweite Aufnahmeelement jeweils an der Lateralschiene und an der Medialschiene befestigt. Bevorzugt sind die beiden Aufnahmeelemente dabei jeweils schwenkbar an den beiden Schienen angeordnet. Zusätzlich bleiben die Aufnahmeelemente jedoch auch aneinander befestigt, wobei dies in unterschiedlichen Positionen geschehen kann, die zu den bereits beschriebenen unterschiedlichen Kniefrontalwinkeln führen.

Vorteilhafterweise verfügt das erste Aufnahmeelement und das zweite Aufnahmeelement über jeweils wenigstens ein Formschlusselement, die korrespondierend ausgebildet sind. Auf diese Weise ist eine Verbindung und Festlegung der beiden Aufnahmeelemente relativ zueinander und aneinander auf besonders einfache Weise möglich.

In einer bevorzugten Ausgestaltung ist wenigstens eines der Formschlusselemente eine Kulisse, beispielsweise ein gebogenes Langloch, während das andere Formschlusselement in dieser Kulisse beweglich gelagert ist, wobei vorzugsweise die Kulisse derart ausgebildet ist, dass eine Orientierung des ersten Aufnahmeelementes relativ zu dem zweiten Aufnahmeelementes konstant bleibt. Das andere Formschlusselement kann beispielsweise ein Stift oder Bolzen sein, der von dem jeweiligen Aufnahmeelement hervorsteht und durch die Kulisse hindurchragt. Das Formschlusselement in Form des Stiftes ist in der Kulisse verschieblich gelagert, wodurch unterschiedliche Positionen der beiden Aufnahmeelemente relativ zueinander realisiert werden. In einer besonders bevorzugten Ausgestaltung ist die Kulisse in Form eines Langloches ausgebildet, das kreisbogenförmig verläuft.

Ist die Kulisse derart ausgebildet, dass eine Orientierung der beiden Aufnahmeelemente zueinander, also ein Winkel zwischen den beiden, konstant bleibt, wird dadurch erreicht, dass auch der Winkel zwischen den Bereichen des Unterschenkelteiles und/oder des Oberschenkelteiles konstant bleibt, an denen die beiden Aufnahmeelemente angeordnet sind. Dabei handelt es sich vorzugsweise um Medialschienen und Lateralschienen. Bleiben diese unabhängig von der Position, in der die beiden Aufnahmeelemente aneinander angeordnet werden, parallel, bleibt eine einmal eingestellte Passform und der damit verbundene Tragekomfort erhalten. Eine solche Bewegung der beiden Aufnahmeelemente wird kollineare Bewegung genannt. Der Winkel bleibt vorzugsweise also auch dann konstant, wenn sich der Kniefrontalwinkel ändert.

Die Arretierungsvorrichtung ist in diesem Fall vorteilhafterweise eine Schraubverbindung. Das andere Formschlusselement, das in der Kulisse verschieblich gelagert ist, kann beispielsweise mit einem Außengewinde versehen sein, auf das eine Mutter aufsetzbar ist. Durch Anziehen der Mutter wird das die Kulisse tragende Aufnahmeelement zwischen dem anderen Aufnahmeelement und der Mutter eingeklemmt, wodurch es zu einer Festlegung und Arretierung kommt. Selbstverständlich können hier unterschiedliche Unterlegscheiben oder Zwischenlegelemente vorhanden sein, um die auftretenden Druckbelastungen möglichst optimal zu verteilen.

Vorzugsweise verfügt das erste Aufnahmeelement und/oder das zweite Aufnahmeelement über mehrere Formschlusselemente, die versetzt zueinander angeordnet sind

Vorzugsweise ist die wenigstens eine Arretierungseinrichtung eingerichtet, das erste Aufnahmeelement am zweiten Aufnahmeelement oder umgekehrt festzulegen. Dies kann, muss jedoch nicht, über die bereits beschriebenen Formschlusselemente geschehen.

Vorzugsweise ist die Weite des Oberschenkelteils der Knieorthese einstellbar. Dazu sind beispielsweise mehrere Kulissen vorhanden, die vorzugsweise parallel zueinander verlaufen. Das in der Kulisse verschiebbare Formschlusselement ist dabei beispielsweise durch ein Loch geführt, das in dem Aufnahmeelement angeordnet ist, das nicht die Kulissen aufweist. Das Formschlusselement kann in die unterschiedlichen Kulissen geführt sein, wodurch sich die Weite des Oberschenkelteils ändert. Alternativ oder zusätzlich dazu können auch mehrere Löcher in dem jeweiligen Aufnahmeelement vorhanden sein, durch die das Formschlusselement geführt werden kann.

In einer bevorzugten Ausgestaltung ist die Arretierungseinrichtung in Form wenigstens eines Klettverschlusselementes und wenigstens eines Klettverschlussgegenelementes ausgebildet. Das Klettverschlusselement ist vorteilhafterweise an einem der Aufnahmeelemente, also am ersten Aufnahmeelement oder am zweiten Aufnahmeelement angeordnet, während das Klettverschlussgegenelement vorteilhafterweise am jeweils anderen Aufnahmeelement angeordnet ist. Eine derartig ausgebildete Arretierungseinrichtung kann zusätzlich oder alternativ zu den bereits beschriebenen Formschlusselementen vorhanden sein. So ist es beispielsweise möglich, die Aufnahmeelemente mit den bereits beschriebenen Formschlusselementen auszustatten und zusätzlich eine Arretierungseinrichtung mit Klettverschluss- und Klettverschlussgegenelementen auszubilden.

Erfindungsgemäß verfügt die Lateralschiene und/oder die Medialschiene über wenigstens zwei Bauteile, die durch wenigstens ein Frontalgelenk miteinander verbunden sind. Ein Frontalgelenk verfügt über eine Schwenkachse, die senkrecht zur Schwenkachse des Schwenkgelenkes und damit vorzugsweise auch senkrecht zur Kniegelenksachse verläuft. Ein Frontalgelenk heißt dabei Frontalgelenk, weil seine Schwenkachse senkrecht zu einer Frontalebene verläuft und somit die beiden durch das Frontalgelenk verbundenen Bauteile in einer Frontalebene gegeneinander verschwenkt werden können. Die Frontalgelenke sind beispielsweise in Form von Scharnieren, beispielsweise in Form von Filmscharnieren, ausgebildet.

Durch diese Verschwenkbarkeit der wenigstens zwei Bauteile der Lateralschiene und/oder der Medialschiene können die Medialschiene und die Lateralschiene den unterschiedlichen Positionen, in denen die beiden Aufnahmeelemente zueinander befestigbar sind, Rechnung tragen und einer gegebenenfalls gewollten Bewegung der Bauteile relativ zueinander folgen.

Vorteilhafterweise ist die Arretierungseinrichtung eingerichtet, das Frontalgelenk festzulegen. Durch die Festlegung wenigstens eines Frontalgelenkes, vorteilhafterweise jedoch beider Frontalgelenke, ist eine weitere Verschiebung und eine Änderung der Position des lateralen Aufnahmeelementes relativ zum medialen Aufnahmeelement nicht mehr möglich, sodass auch eine derartig ausgebildete Arretierungseinrichtung die beiden Elemente relativ zueinander festlegt, ohne dass die Arretierungseinrichtung dazu an einem oder beiden der Aufnahmeelemente angeordnet sein muss. Arretierungseinrichtungen, die eine Bewegung eines Gelenkes blockieren, sind aus dem Stand der Technik seit langem bekannt. Vorteilhafterweise verfügt das Frontalgelenk, vorteilhafterweise jedes Frontalgelenk, über eine Einstelleinrichtung, mit der eine Winkelstellung des Frontalgelenkes einstellbar ist. Auf diese Weise lässt sich insbesondere eine reproduzierbare und leicht einstellbare Winkelstellung erreichen.

Alternativ oder zusätzlich dazu ist die Medialschiene und/oder die Lateralschiene elastisch ausgebildet. Sie kann beispielsweise aus einem Metall, insbesondere einem Federstahl, hergestellt sein und durch eine Verschiebung der beiden Aufnahmeelemente relativ zueinander in eine andere Position vorgespannt werden. Auch dadurch lässt sich ein Winkel zwischen dem Unterschenkelteil und dem Oberschenkelteil den jeweiligen körperlichen Gegebenheiten anpassen und auch eine auf das Knie bzw. auf den Oberschenkel und den Unterschenkel ausübbare Kraft anpassen.

Vorteilhafterweise sind die Medialschiene und die Lateralschiene nicht längenveränderlich ausgebildet.

An dem Oberschenkelteil und/oder dem Unterschenkelteil ist wenigstens ein, bevorzugt jeweils wenigstens ein Befestigungselement angeordnet, mittels dessen die Knieorthese an dem Oberschenkel und/oder dem Unterschenkel des Trägers befestigbar ist. Vorzugsweise handelt es sich dabei um Gurte, die mit entsprechenden Verschlusselementen versehen sind. Die Gurte sind bevorzugt über Laschen an dem jeweiligen Bauteil befestigt. Andere Befestigungselemente, beispielsweise Riemen, Bänder oder Zugsysteme, wie beispielsweise das von der Firma BOA Technology Inc. angeordnete Zugsystem sind zusätzlich oder alternativ einsetzbar. Das Zugsystem der BOA Technology Inc. Hat den Vorteil, dass aufgrund der einfachen Einstellbarkeit der Länge des eigentlichen Zugelementes die Orthese leicht angelegt und individuell angepasst werden kann.

Mit Hilfe der beiliegenden Zeichnungen wird nachfolgend ein Ausführungsbeispiel der vorliegenden Erfindung näher erläutert. Es zeigen:
- Figuren 1 bis 8: Eine Knieorthese gemäß einem Ausführungsbeispiel der vorliegenden Erfindung in unterschiedlichen Positionen,
- Figuren 9 bis 11: eine Knieorthese gemäß einem weiteren Ausführungsbeispiel der vorliegenden Erfindung.

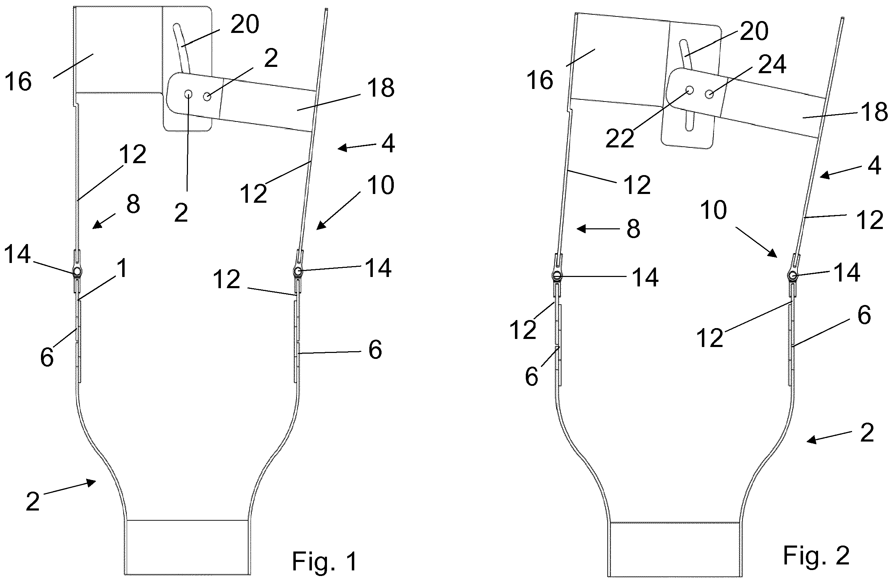

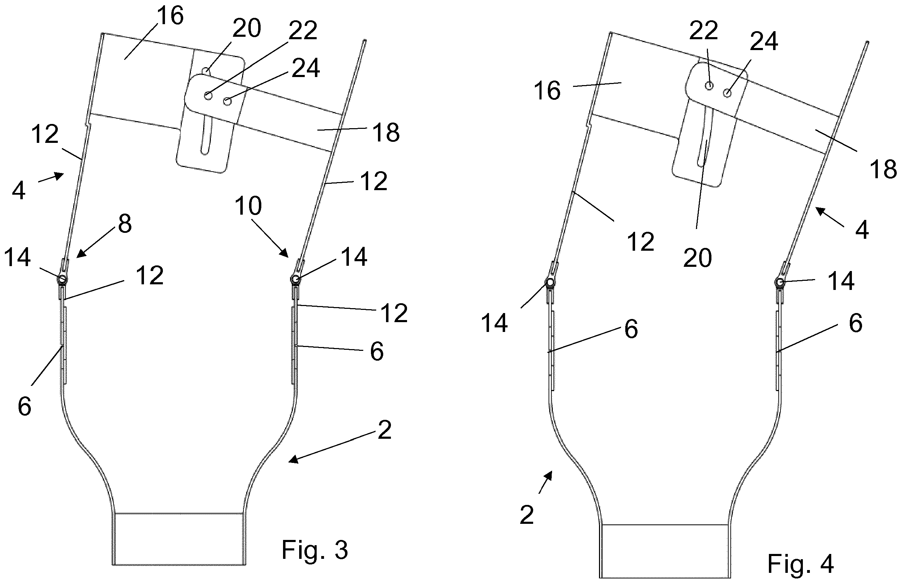

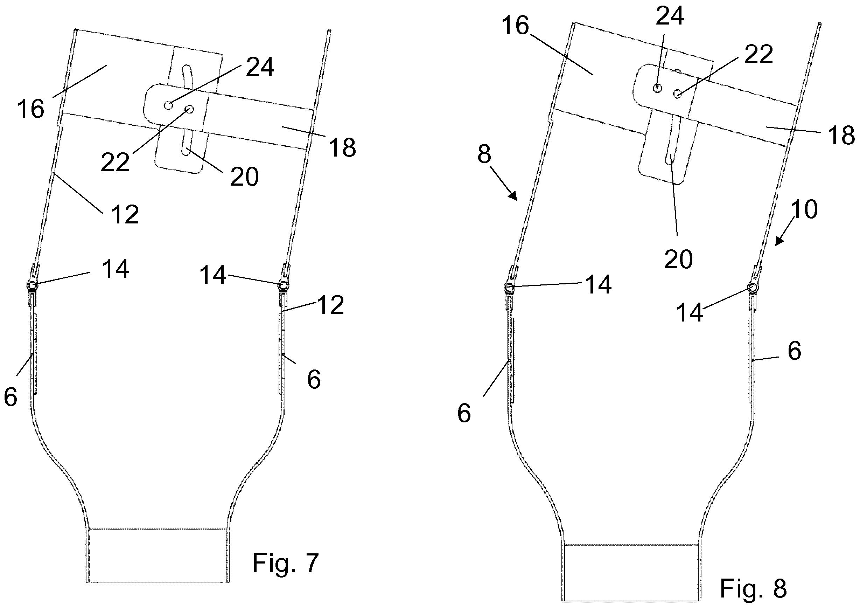

Die in den Figuren 1 bis 8 gezeigte Knieorthese verfügt über ein Unterschenkelteil 2 und ein Oberschenkelteil 4, die im gezeigten Ausführungsbeispiel durch zwei Schwenkgelenke 6 miteinander verbunden sind. Das Unterschenkelteil 2 wird im angelegten Zustand der Knieorthese am Unterschenkel des Trägers und das Oberschenkelteil 4 am Oberschenkel des Trägers angeordnet. Die Schwenkgelenke 6 erlauben eine Verschwenkung des Unterschenkelteils 2 relativ zum Oberschenkelteil 4 und sind vorzugsweise so ausgerichtet, dass die Schwenkachse der beiden Schwenkgelenke 6 mit der Knieachse des Trägers zusammenfällt.

Im gezeigten Ausführungsbeispiel verfügt das Oberschenkelteil 4 über eine Lateralschiene 8 und eine Medialschiene 10, die im bezeigten Ausführungsbeispiel jeweils zwei Bauteile 12 aufweisen, die jeweils durch ein Frontalgelenk 14 schwenkbar miteinander verbunden sind.

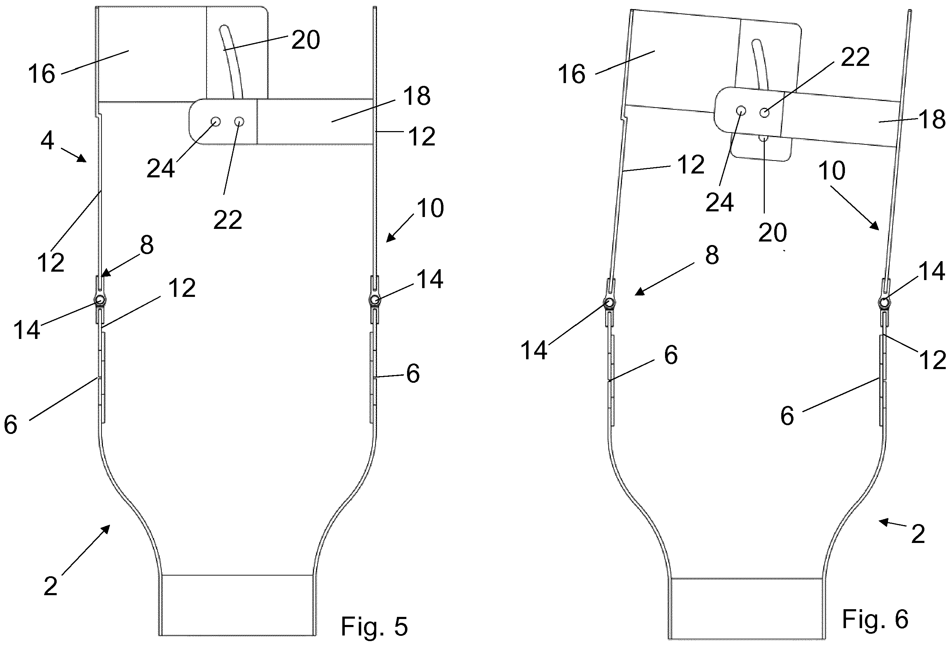

Die Lateralschiene 8 verfügt zudem über ein erstes Aufnahmeelement 16 und die Medialschiene 10 verfügt über ein zweites Aufnahmeelement 18. Das erste Aufnahmeelement 16 ist an der Lateralschiene 8 angeordnet und verfügt über ein Formschlusselement in Form einer Kulisse 20, in der ein zweites Formschlusselement in Form eines Stiftes 22, das am zweiten Aufnahmeelement 18 angeordnet ist, verschieblich angeordnet ist. Das zweite Aufnahmeelement 18 ist an der Medialschiene 10 angeordnet. Der Stift 22 ist in einer von im gezeigten Ausführungsbeispiel zwei vorhandenen Löchern 24 angeordnet und durch die Kulisse 20 geführt. Dadurch ist es möglich, eine Weite des Oberschenkelteils 4 einstellbar auszugestalten. Die Figuren 5 bis 8 zeigen die Knieorthese, wobei nun der Stift 22 in dem zweiten der beiden Löcher 24 angeordnet ist. Selbstverständlich können auch mehr als zwei Löcher 24 vorhanden sein, um die Weite des Oberschenkelteils 4 in mehreren Stufen einstellbar zu gestalten. Zusätzlich oder alternativ dazu können auch mehrere Kulissen 22 vorhanden sein, die vorzugsweise parallel zueinander verlaufen. Auch dadurch wird die Weite des Oberschenkelteils 4 einstellbar.

Die Figuren 1 bis 4 zeigen die gleiche Knieorthese, wobei der Stift 22 in unterschiedlichen Positionen in der Kulisse 20 angeordnet ist. Dadurch kommt es zum Verschwenken der beiden Frontalgelenke 14, sodass ein Winkel zwischen dem Oberschenkelteil 4 und dem Unterschenkelteil 2 verändert wird. Über eine nicht dargestellte Arretierungseinrichtung kann beispielsweise das erste Aufnahmeelement 16 am zweiten Aufnahmeelement 18 festgelegt werden. Alternativ oder zusätzlich dazu kann eine Arretierungsvorrichtung vorhanden sein, durch die ein weiteres Verschwenken eines der Frontalgelenke 14 unterbunden wird.

Man erkennt in den Figuren, dass die Medialschiene 10 und die Lateralschiene 8 in jeder Stellung parallel zueinander verlaufen, so dass sich der Winkel zwischen beiden nicht ändert. Dies wird durch die besondere Form der Kulisse 20 erreicht, indem auch eine Orientierung der beiden Aufnahmeelemente (16,18) zueinander konstant bleibt. Die Verschiebung der beiden Aufnahmeelemente erfolgt kollinear.

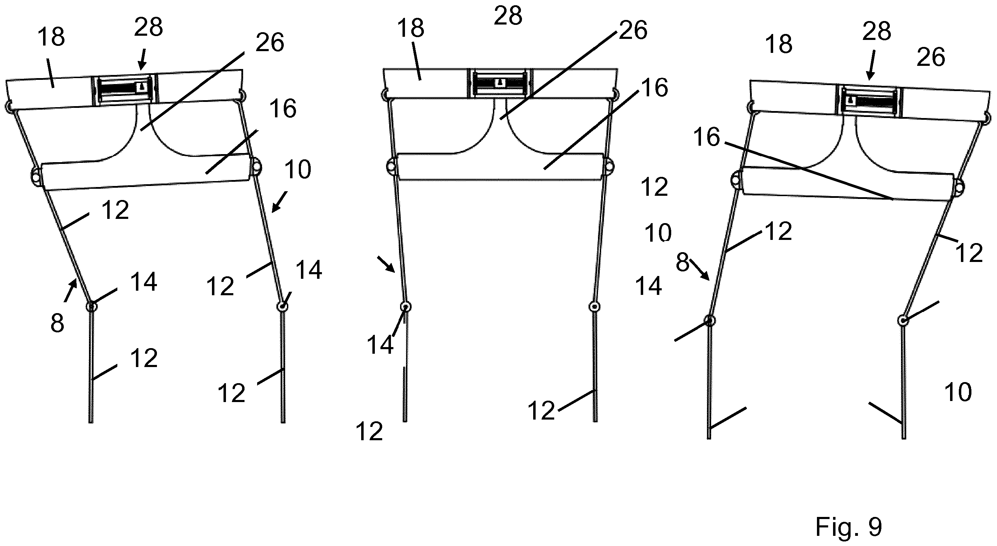

Figur 9 zeigt einen Teil einer Knieorthese gemäß einem weiteren Ausführungsbeispiel der vorliegenden Erfindung. Dargestellt ist dabei jeweils nur der Oberschenkelteil 4. In jeder der drei Darstellungen der Figur 9 ist der Oberschenkelteil 4 mit einer Lateralschiene 8 und einer Medialschiene 10 dargestellt, die jeweils ein Frontalgelenk 14 aufweisen, durch das jeweils zwei Bauteile 12 miteinander verbunden sind. Zwischen den Bauteilen 12, die oberhalb der jeweiligen Frontalgelenke 14 dargestellt sind, ist das erste Aufnahmeelement 16 und das zweite Aufnahmeelement 18 zu erkennen. Anders als beim bisher gezeigten Ausführungsbeispiel sind sowohl das erste Aufnahmeelement 16 als auch das zweite Aufnahmeelement 18 sowohl an der Lateralschiene 8 als auch an der Medialschiene 10 angeordnet. Dabei sind die Verbindungen zwischen jeweils zwei dieser Bauteile Schwenkverbindungen, wie in den unterschiedlichen Darstellungen der Figur 9 deutlich zu erkennen ist.

Das erste Aufnahmeelement 16 verfügt über einen sich nach oben erstreckenden Vorsprung 26, mit dem es in eine Arretierungseinrichtung 28 eingreift. Man erkennt, dass in der linken Darstellung der Figur 9 der Vorsprung 26 im rechten Teil der Arretierungseinrichtung 28 angeordnet ist, während er in der linken Darstellung der Figur 9 im linken Teil der Arretierungseinrichtung und in der mittleren Teil der Darstellung im mittleren Teil der Arretierungseinrichtung angeordnet ist. Dadurch verändert sich nicht nur die Position des ersten Aufnahmeelementes 16 relativ zum zweiten Aufnahmeelement 18, sondern auch der Winkel zwischen den beiden Bauteilen 12, die durch die beiden Aufnahmeelemente 16, 18, verbunden sind und damit auch ein Kniefrontalwinkel der Knieorthese.

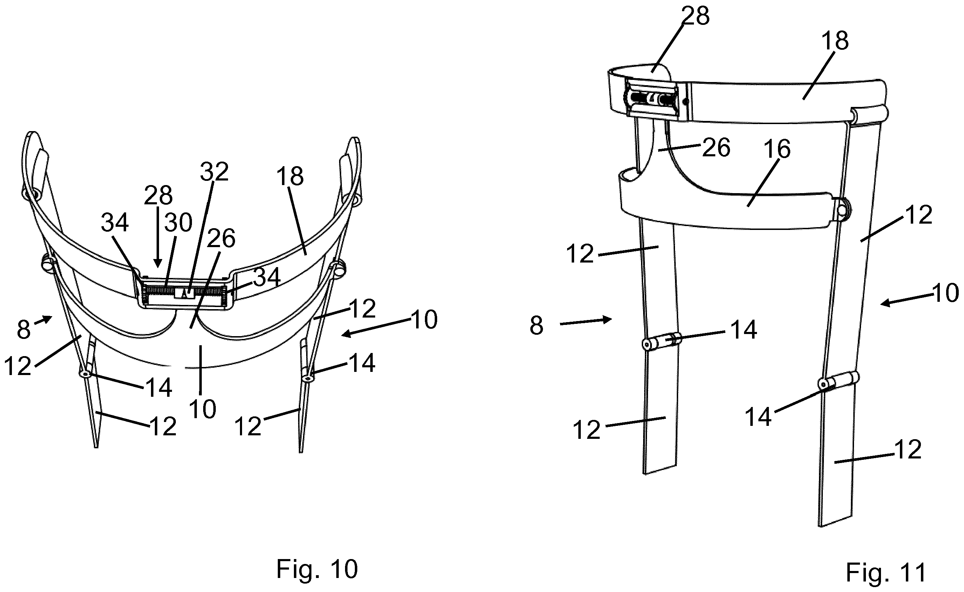

Figur 10 zeigt den Teil der Vorrichtung aus Figur 9 in einer Darstellung schräg von oben. Man erkennt das erste Aufnahmeelement 16 mit dem Vorsprung 26 sowie das zweite Aufnahmeelement 18, die jeweils beide mit den Bauteilen 12 verbunden sind, die über Frontalgelenke 14 mit weiteren Bauteilen verbunden sind und gemeinsam mit diesen die Lateralschiene 8 und die Medialschiene 10 bilden. In dieser Darstellung ist die Arretierungseinrichtung 28 besonders detailliert zu erkennen. Sie verfügt über eine Gewindestange 30, die mit einem Innengewinde an einem Anschlussstück 32, das am Vorsprung 26 angeordnet ist, zusammenwirkt. Über zwei Rändelräder 34 ist die Gewindestange 30 drehbar, wodurch das Anschlussstück 32 und damit auch der Vorsprung 26 und das erste Aufnahmeelement 16 relativ zur Arretierungseinrichtung 28 und damit auch relativ zum zweiten Aufnahmeelement 18 verschoben wird, wodurch die drei in Figur 9 dargestellten Positionen sowie alle Zwischenpositionen erreicht werden können.

Figur 11 zeigt die Darstellung aus Figur 10 in einer anderen Ansicht. Man erkennt wieder die Bauteile 12, die die Medialschiene 10 und die Lateralschiene 8 bilden und jeweils durch ein Frontalgelenk 14 miteinander verbunden sind. Das erste Aufnahmeelement 16 und das zweite Aufnahmeelement 18 sind jeweils an der Medialschiene 10 und an der Lateralschiene 8 befestigt.

Um die Schwenkbarkeit der Vorrichtung in die in Figur 9 gezeigte Position zu gewährleisten, ist es von Vorteil, wenn die Drehpunkte, an denen die unterschiedlichen Bauteile miteinander verbunden sind, zumindest auf einer Seite variabel, beispielsweise in Form eines Langloches, ausgebildet sind. Dies gilt insbesondere für zumindest eine Befestigung des ersten Aufnahmeelementes 16 an einem der Bauteile 12. Vorzugsweise ist auch die Arretierungseinrichtung 28 oder zumindest ein Teil davon, beispielsweise die Gewindestange 30, die ohnehin drehbar ausgebildet ist, auf einer Seite mit einem variablen Drehpunkt ausgestaltet. Dies ist vorteilhafterweise die gleiche Seite, auf der auch der Drehpunkt zwischen dem ersten Aufnahmeelement 16 und dem jeweiligen Bauteil 12 als variabler Drehpunkt ausgebildet ist.

### Bezugszeichenliste

- 2: Unterschenkelteil
- 4: Oberschenkelteil
- 6: Schwenkgelenk
- 8: Lateralschiene
- 10: Medialschiene
- 12: Bauteil
- 14: Frontalgelenk
- 16: erstes Aufnahmeelement
- 18: zweites Aufnahmeelement
- 20: Kulisse
- 22: Stift
- 24: Loch
- 26: Vorsprung
- 28: Arretierungseinrichtung
- 30: Gewindestange
- 32: Anschlussstück
- 34: Rändelrad

## Patentansprüche

1. Knieorthese mit
einem Unterschenkelteil (2), das sich in einer Unterschenkelrichtung erstreckt, und
einem Oberschenkelteil (4), das sich in einer Oberschenkelrichtung erstreckt, und
wenigstens einer Arretierungseinrichtung,
wobei das Unterschenkelteil (2) und das Oberschenkelteil (4) durch wenigstens ein Schwenkgelenk (6) schwenkbar mit einander verbunden sind,
das Unterschenkelteil (2) und/oder das Oberschenkelteil (4) eine Lateralschiene (8) und eine Medialschiene (10) aufweist, und
die Unterschenkelrichtung mit der Oberschenkelrichtung einen Kniefrontalwinkel einschließt,
wobei
die Knieorthese ein erstes Aufnahmeelement (16) und ein zweites Aufnahmeelement (18) aufweist, die in verschiedenen Positionen relativ zueinander mittels der wenigstens einen Arretierungseinrichtung (28) festlegbar sind, wobei die Lateralschiene (8) und/oder die Medialschiene (10) wenigstens zwei Bauteile (12) aufweist, die durch ein Frontalgelenk (14) miteinander verbunden sind, **dadurch gekennzeichnet, dass** die verschiedenen Positionen des ersten Aufnahmeelementes (16) und des zweiten Aufnahmeelementes (18) zu unterschiedlichen Kniefrontalwinkeln zwischen der Unterschenkelrichtung und der Oberschenkelrichtung führen.

2. Knieorthese nach Anspruch 1, **dadurch gekennzeichnet, dass** das erste Aufnahmeelement (16) an der Lateralschiene (8) und das zweite Aufnahmeelement (18) an der Medialschiene (10) befestigt ist.

3. Knieorthese nach Anspruch 2, **dadurch gekennzeichnet, dass** das erste Aufnahmeelement (16) und das zweite Aufnahmeelement (18) jeweils an der Lateralschiene (8) und an der Medialschiene (10) befestigt, vorzugsweise schwenkbar befestigt ist.

4. Knieorthese nach einem der vorstehenden Ansprüche, **dadurch gekennzeichnet, dass** das erste Aufnahmeelement (16) und das zweite Aufnahmeelement (18) jeweils wenigstens ein Formschlusselement aufweisen, die korrespondierend ausgebildet sind.

5. Knieorthese nach Anspruch 4, **dadurch gekennzeichnet, dass** wenigstens eines der Formschlusselemente eine Kulisse (20) ist, in der das andere Formschlusselement (22) beweglich gelagert ist, wobei vorzugsweise die Kulisse (20) derart ausgebildet ist, dass eine Orientierung des ersten Aufnahmeelementes (16) relativ zu dem zweiten Aufnahmeelementes (18) konstant bleibt.

6. Knieorthese nach Anspruch 5, **dadurch gekennzeichnet, dass** das erste Aufnahmeelement (16) und/oder das zweite Aufnahmeelement (18) mehrere Formschlusselemente aufweist, die versetzt zueinander angeordnet sind.

7. Knieorthese nach einem der vorstehenden Ansprüche, **dadurch gekennzeichnet, dass** die wenigstens eine Arretierungseinrichtung eingerichtet ist, das erste Aufnahmeelement (16) am zweiten Aufnahmeelement (18) oder umgekehrt festzulegen.

8. Knieorthese nach Anspruch 7, **dadurch gekennzeichnet, dass** die Arretierungseinrichtung in Form wenigstens eines Klettverschlusselementes, das vorzugsweise an einem der Aufnahmeelemente (16, 18) angeordnet ist, und wenigstens eines Klettverschlussgegenelementes ausgebildet ist, das vorzugsweise an dem anderen Aufnahmeelement (18, 16) angeordnet ist.

9. Knieorthese nach einem der vorstehenden Ansprüche, **dadurch gekennzeichnet, dass** die wenigstens eine Arretierungseinrichtung eingerichtet ist, das Frontalgelenk (14) festzulegen.

10. Knieorthese nach einem der vorstehenden Ansprüche, **dadurch gekennzeichnet, dass** das Frontalgelenk (14) eine Einstelleinrichtung aufweist, mit einer Winkelstellung des Frontalgelenks (14) einstellbar ist.

11. Knieorthese nach einem der vorstehenden Ansprüche, **dadurch gekennzeichnet, dass** die Medialschiene (10) und/oder die Lateralschiene (8) elastisch, beispielsweise aus einem Metall, insbesondere einem Federstahl, ausgebildet sind.

12. Knieorthese nach einem der vorstehenden Ansprüche, **dadurch gekennzeichnet, dass** das die Medialschiene (10) und die Lateralschiene (8) nicht längenveränderlich ausgebildet sind.

## Claims

1. A knee orthosis with
a lower leg part (2) that extends in a lower leg direction, and
an upper leg part (4) that extends in a upper leg direction, and
at least one locking device,
wherein the lower leg part (2) and the upper leg part (4) are connected to each other by at least one swivel joint (6) such that they can be swivelled, the lower leg part (2) and/or the upper leg part (4) comprise a lateral splint (8) and a medial splint (10), and
the lower leg direction encloses a knee frontal angle with the upper leg direction,
wherein the knee orthosis has a first accommodation element (16) and a second accommodation element (18) which can be fixed in different positions relative to each other via the at least one locking device (28), wherein the lateral splint (8) and/or the medial splint (10) comprise at least two components (12), which are connected to each other by a frontal joint (14),
**characterized in that** the different positions of the first accommodation element (16) and the second accommodation element (18) lead to different knee frontal angles between the lower leg direction and the upper leg direction.

2. The knee orthosis according to claim 1, **characterized in that** the first accommodation element (16) is attached to the lateral splint (8) and the second accommodation element (18) to the medial splint (10).

3. The knee orthosis according to claim 2, **characterized in that** the first accommodation element (16) and the second accommodation element are attached to the lateral splint (8) and the medial splint (10) respectively, preferably such that they can be swivelled.

4. The knee orthosis according to one of the preceding claims, **characterized in that** the first accommodation element (16) and the second accommodation element (18) each comprise at least one positive-locking element, which are designed to correspond with one another.

5. The knee orthosis according to claim 4, **characterized in that** at least one of the positive-locking elements is a slot (20), in which the other positive-locking element (22) is mounted such that it can be moved, the slot (20) preferably being designed in such a way that an orientation of the first accommodation element (16) relative to the second accommodation element (18) remains constant.

6. The knee orthosis according to claim 5, **characterized in that** the first accommodation element (16) and/or the second accommodation element (18) comprises multiple positive-locking elements arranged at an offset to each other.

7. The knee orthosis according to one of the preceding claims, **characterized in that** the at least one locking device is configured to fix the first accommodation element (16) to the second accommodation element (18) or vice-versa.

8. The knee orthosis according to claim 7, **characterized in that** the locking device is designed in the form of a velcro element, preferably arranged on one of the accommodation elements (16, 18), and at least one velcro counter-element, preferably arranged on the other accommodation element (18, 16).

9. The knee orthosis according to claim one of the preceding claims, **characterized in that** the least one locking device is configured to fix the frontal joint (14).

10. The knee orthosis according to one of the preceding claims, **characterized in that** the frontal joint (14) features an adjustment device with which an angular position of the frontal joint (14) can be adjusted.

11. The knee orthosis according to one of the preceding claims, **characterized in that** the medial splint (10) and/or the lateral splint (8) are designed to be elastic, for example made from a metal, especially a spring steel.

12. The knee orthosis according to one of the preceding claims, **characterized in that** the medial splint (10) and/or the lateral splint (8) are designed such that they are not variable in length.

## Revendications

1. Orthèse de genou, comprenant
une partie de jambe (2) qui s'étend dans une direction de la jambe, et
une partie de cuisse (4) qui s'étend dans une direction de la cuisse, et
au moins un dispositif d'arrêt,
dans laquelle
la partie de jambe (2) et la partie de cuisse (4) sont reliées entre elles de manière à pouvoir pivoter par l'intermédiaire d'au moins une articulation pivotante (6),
la partie de jambe (2) et/ou la partie de cuisse (4) présente(nt) un rail latéral (8) et un rail médian (10), et
la direction de la jambe forme un angle frontal du genou avec la direction de la cuisse,
l'orthèse de genou comprend un premier élément de réception (16) et un deuxième élément de réception (18) aptes à être fixés l'un par rapport à l'autre dans différentes positions au moyen dudit au moins un dispositif d'arrêt (28),
le rail latéral (8) et/ou le rail médian (10) présente(nt) au moins deux composants (12), qui sont reliés entre eux par une articulation frontale (14), **caractérisée en ce que** les différentes positions du premier élément de réception (16) et du deuxième élément de réception (18) assurent des angles frontaux différents du genou entre la direction de la jambe et la direction de la cuisse.

2. Orthèse de genou selon la revendication 1,
**caractérisée en ce que** le premier élément de réception (16) est fixé au rail latéral (8) et le deuxième élément de réception (18) est fixé au rail médian (10).

3. Orthèse de genou selon la revendication 2,
**caractérisée en ce que** le premier élément de réception (16) et le deuxième élément de réception (18) sont chacun fixés au rail latéral (8) et au rail médian (10), de préférence de manière à pouvoir pivoter.

4. Orthèse de genou selon l'une des revendications précédentes,
**caractérisée en ce que** le premier élément de réception (16) et le deuxième élément de réception (18) présentent chacun au moins un élément de coopération de forme, qui sont réalisés de manière correspondante.

5. Orthèse de genou selon la revendication 4,
**caractérisée en ce que** l'un au moins des éléments de coopération de forme est une coulisse (20) dans laquelle l'autre élément de coopération de forme (22) est logé de manière mobile, de préférence, la coulisse (20) étant conçue de telle sorte qu'une orientation du premier élément de réception (16) par rapport au deuxième élément de réception (18) reste constante.

6. Orthèse de genou selon la revendication 5,
**caractérisée en ce que** le premier élément de réception (16) et/ou le deuxième élément de réception (18) présente(nt) plusieurs éléments de coopération de forme qui sont décalés les uns par rapport aux autres.

7. Orthèse de genou selon l'une des revendications précédentes,
**caractérisée en ce que** ledit au moins un dispositif d'arrêt est agencé pour fixer le premier élément de réception (16) au deuxième élément de réception (18) ou inversement.

8. Orthèse de genou selon la revendication 7,
**caractérisée en ce que** le dispositif d'arrêt se présente sous la forme d'au moins un élément de fermeture auto-agrippante, disposé de préférence sur l'un des éléments de réception (16, 18), et d'au moins un contre-élément de fermeture auto-agrippante disposé de préférence sur l'autre élément de réception (18, 16).

9. Orthèse de genou selon l'une des revendications précédentes,
**caractérisée en ce que** ledit au moins un dispositif d'arrêt est agencé pour fixer l'articulation frontale (14).

10. Orthèse de genou selon l'une des revendications précédentes,
**caractérisée en ce que** l'articulation frontale (14) présente un dispositif de réglage permettant de régler une position angulaire de l'articulation frontale (14).

11. Orthèse de genou selon l'une des revendications précédentes,
**caractérisée en ce que** le rail médian (10) et/ou le rail latéral (8) sont réalisés de manière élastique, par exemple en un métal, en particulier en un acier à ressort.

12. Orthèse de genou selon l'une des revendications précédentes,
**caractérisée en ce que** le rail médian (10) et le rail latéral (8) ne sont pas de longueur variable.
